# EUROPÄISCHE PATENTSCHRIFT

(11) **EP 1 188 054 B1**
(45) Veröffentlichungstag und Bekanntmachung des Hinweises auf die Patenterteilung: **13.08.2003**
(21) Anmeldenummer: 00927221.2
(22) Anmeldetag: 19.05.2000
(51) Int. Cl.: G01N 33/36, G01N 1/28

(54) **VERFAHREN UND VORRICHTUNG ZUR MESSUNG ORGANISCHER UND ANORGANISCHER BEGLEITFRACHTEN IN EINEM FLÄCHIGEN GUT**
METHOD AND DEVICE FOR MEASURING ORGANIC AND INORGANIC ACCOMPANYING SUBSTANCES IN A FLAT ITEM
PROCEDE ET DISPOSITIF POUR LA MESURE DE SUBSTANCES D'ACCOMPAGNEMENT ORGANIQUES ET INORGANIQUES DANS UN PRODUIT PLAT

(30) Priorität: 21.05.1999 DE 19923461
(43) Veröffentlichungstag der Anmeldung: 20.03.2002
(73) Patentinhaber: Mahlo GmbH & Co. KG, 93342 Saal (DE)
(72) Erfinder: RAUSCH, Oswald, D-93356 Teugn (DE); ENGELMEIER-STROER, Hermann, D-93346 Ihrlerstein (DE)
(74) Vertreter: Bohnenberger, Johannes, Dr.
(86) Internationale Anmeldenummer: EP0004537
(87) Internationale Veröffentlichungsnummer: WO00072006

(56) Entgegenhaltungen:
- EP-A1- 0 640 825
- DE-A1- 4 324 491
- US-A- 3 986 274

## Beschreibung

Die Erfindung betrifft ein Verfahren zur Messung organischer und/oder anorganischer Begleitfrachten im Inneren eines flüssigkeitsdurchlässigen flächigen Gutes, insbesondere eines Textilgutes, nach dem Oberbegriff des Anspruches 1 sowie eine Vorrichtung zur Durchführung dieses Verfahrens.

Bei der Verarbeitung von Textilgut, insbesondere bei Färbeoder Veredelungsschritten, muß der Gehalt an Fremdstoffen aus vorausgehenden Behandlungen (Waschen, gegebenenfalls Absäuern, Abquetschen, Trocknen) bekannt sein, die einen Einfluß auf die nachfolgenden Behandlungsschritte haben können. Es ist daher erforderlich, das Vorhandensein und möglichst auch den Gehalt solcher Fremdstoffe - nachfolgend als organische und anorganische Begleitfrachten bezeichnet - zu bestimmen.

Aus der DD 223 535 A1 ist ein Verfahren bekannt,-bei dem nach dem Auslaufen einer Textilbahn aus einer Reinigungsflotte zunächst der Hauptteil der aufgenommenen Flüssigkeit durch Abquetschen entfernt und nach einem vorbestimmten Zeitraum eine weitere Flüssigkeitsmenge entzogen wird. Die letztere wird gesammelt und auf die in ihr enthaltene Konzentration an Begleitfrachten untersucht. Dieses Verfahren hat insofern Grenzen, als bei starkem Abquetschen auf einen geringen Restfeuchtegehalt nur noch schwer eine Probenahme für die Konzentrationsmessung möglich ist. Will man andererseits in jedem Fall eine erfolgreiche Probenahme sichern, muß der Restfeuchtegehalt des gesamten Textilgutes nach dem Abquetschen allein aus meßtechnischen Gründen relativ hoch gehalten werden, was natürlich den Energieaufwand bei einer anschließenden Trocknung erhöht.

Aus der DE 41 31 616 C2 bzw. DE 43 24 491 C2 ist ein Verfahren der gattungsgemäßen Art sowie eine Vorrichtung zur Durchführung dieses Verfahrens bekannt. Hierbei werden nach dem Abquetschen aus dem Textilgut abschnittsweise Probemengen der Begleitfracht durch Aufbringen und anschließendes Absammeln von Lösungsmittel auf Abschnitte des Textilgutes entnommen. Die im abgesammelten Lösungsmittel enthaltene Begleitfracht wird dann quantitativ und qualitativ bestimmt. Das Lösungsmittel durchspült insbesondere das Innere des flächigen Textilgutes senkrecht zu dessen Oberfläche und nimmt die an das Textilmaterial gebundenen oder dispergierten Begleitfrachten mit.

Bei diesem bekannten Verfahren wird das Textilgut an der Meßstelle zwischen zwei Metallplatten mit sehr geringem Abstand hindurchgeführt, von denen die eine mit mindestens einer Düse zum Aufsprühen des Lösungsmittels und die andere mit mindestens einer Absaugöffnung zum Absammeln des Lösungsmittels versehen ist. Zur Aufrechterhaltung des nötigen Unterdrucks ist der Abstand der Platten sehr gering, was jedoch zum Auftreten einer relativ großen Bremswirkung bei durchlaufendem Textilgut führt. Insbesondere bei elastischer Ware kann diese Bremswirkung zu Verzügen, Faltenbildung, Dehnungen oder ähnlichen nachteiligen Einflüssen führen.

Der Erfindung liegt daher die Aufgabe zugrunde, ein verbessertes Verfahren der gattungsgemäßen Art sowie eine Vorrichtung zu dessen Durchführung anzugeben, bei denen keine nachteiligen mechanischen Veränderungen des Textilgutes auftreten.

Diese Aufgabe wird hinsichtlich ihres Verfahrensaspektes durch ein Verfahren mit den Merkmalen des Anspruches 1 und hinsichtlich ihres Vorrichtungsaspektes durch eine Vorrichtung mit den Merkmalen des Anspruches 4 gelöst.

Die Erfindung schließt den wesentlichen Gedanken ein, die Aufbringung des Lösungsmittels auf das flächige Gut vom Absammeln räumlich zu trennen. Sie schließt weiter den Gedanken ein, das Lösungsmittel nicht in einem engen Spalt, sondern frei, d. h. aus einer mit Abstand zur ersten Obefläche des flächigen Gutes angeordneten Düse, aufzubringen.

Diese Aufbringung des Lösungsmittels erfolgt insbesondere mit einem gewissen Dampfanteil, speziell als Wasser-Wasserdampf-Gemisch.

Bei der entsprechenden Vorrichtung sprüht eine frei über dem durchlaufenden Textilgut angeordnete Düse in Förderrichtung vor der Absaugplatte das Lösungsmittel (speziell ein Gemisch aus heißem Wasserdampf und Wasser) auf das Textilgut. Der räumliche Abstand zur stromabwärts angeordneten Absaugplatte ist ausreichend groß gewählt, um ein Eindringen des Lösungsmittels in die gesamte Dicke des flächigen Gutes zu ermöglichen. Im Bereich der Meßstelle wird das flächige Gut mit kleinem Abstand zur Auflagefläche frei geführt. Der an der Absaugstelle herschende Unterdruck lenkt das Gut zwar in Richtung zur Absaugplatte hin ab, es tritt jedoch keine merkliche Bremswirkung durch die Absaugplatte mehr ein.

Um eine mögliche Rest-Bremswirkung weiter zu minimieren, hat die Absaugplatte eine Oberfläche mit in Bezug auf das durchlaufende Material sehr niedrigem Reibungskoeffizienten. Hierzu besteht die Absaugplatte insbesondere aus Keramik oder weist eine keramische Oberfläche auf.

In einer bevorzugten Ausführung hat die Absaugplatte eine Mehrzahl von in Förderrichtung hintereinander angeordneten und insbesondere schräg zur Förderrichtung ausgerichteten Absaugschlitzen. Diese Schlitzanordnung sichert eine hohe Absaugkapazität, verhindert aber ein streifenförmiges An- bzw. sogar Einsaugen der Textilbahn, wie es bei Vorsehen eines langen, durchgehenden Absaugschlitzes auftreten kann, insbesondere, wenn dieser parallel zur Förderrichtung ausgerichtet ist.

In einer vorteilhaft auf flächiges Gut mit sehr unterschiedlicher Dicke und unterschiedlichen Eigenschaften abstimmbaren Ausführung ist der Abstand zwischen der Aufspritzdüse und der Absaugplatte verstellbar.

In einer weiteren, besonders flexibel auf Textilgut mit unterschiedlichen Eigenschaften sowie auf verschiedene Fördergeschwindigkeiten einstellbaren Ausführung weist die Aufbringeinrichtung Mittel zur Steuerung der Lösungsmittelmenge in Abhängigkeit von der Fördergeschwindigkeit und/oder bestimmten Eigenschaften des flächigen Gutes auf.

Weitere Einstellmöglichkeiten, die die Anwendbarkeit des vorgeschlagenen Verfahrens für ein breites Spektrum von Textiluntersuchungen sicherstellen, bestehen in der Einstellung der Temperatur und/oder des Aufsprühdruckes des eingesetzten Lösungsmittels sowie des Absaug-Unterdruckes an der Absaugplatte. Insbesondere hinsichtlich des letzteren Parameters bietet die bevorzugte Ausführung mit einer Absaugplatte mit einer Mehrzahl schräg angeordneter Schlitze wesentlich erweiterte Einstellmöglichkeiten, da die spezielle Schlitzanordnung die Gefahr eines Einsaugens des Textilgutes auch bei relativ hohem Unterdruck praktisch beseitigt.

Die Absammeleinrichtung weist in einer vorteilhaften Ausführung einen Zyklonabscheider auf, und in einer Fortbildung dieser Ausführung ist das Absauggebläse ein Seitenkanalgebläse, welches über eine Unterdruckleitung mit dem Zyklonabscheider und über diesen schließlich mit der Absaugplatte in Verbindung steht. Der hier als bevorzugt vorgeschlagene Einsatz eines Zyklonabscheiders ermöglicht in vorteilhafter Weise die Nutzung eines hochwirksamen Absauggebläses in Seitenkanalanordnung, ohne daß die Gefahr besteht, daß Komponenten der zu analysierenden Begleitfrachten durch das Absaugen verloren gehen. Speziell in Verbindung mit einem vorgeschalteten Kühler bzw. Kondensator, dessen Einsatz speziell bei Verwendung von dampfförmigem oder einen dampfförmige Komponente umfassendem Lösungsmittel zweckmäßig ist, werden systematische Meßfehler, die durch den Vorgang des Absammelns des belasteten Lösungsmittels bedingt sein können, auf ein Minimum verringert.

Eine besonders energieökonomische Betriebsweise der Vorrichtung wird durch Ausführung des Kühlers als Gegenstromkühler zur gleichzeitigen Vorheizung des aufzusprühenden frischen Lösungsmittels gesichert.

In einer weiteren vorteilhaften Ausführung umfaßt die Vorrichtung Mittel zum Abschalten der Lösungsmittelzufuhr zur Aufspritzdüse sowie zur Verringerung oder Abschaltung des Unterdrucks an der Absaugplatte bei Erfassung eines Stillstandes des durchlaufenden Gutes.

Wie oben bereits erwähnt, sind das vorgeschlagene Verfahren und die Vorrichtung insbesondere zur Erfassung von Fremstoffen bzw. Begleitfrachten in einem Textilgut vorgesehen, grundsätzlich aber auch zur Untersuchung von anderem flächigen Gut, etwa Papier, geeignet.

Vorteile und Zweckmäßigkeiten der Erfindung ergeben sich im übrigen aus den Unteransprüchen sowie der nachfolgenden Beschreibungen von Ausführungsbeispielen anhand der Figuren. Von diesen zeigen:
- Fig. 1: eine schematische Darstellung einer Vorrichtung zur Durchführung des erfindungsgemäßen Verfahrens in einer ersten Ausführungsform,
- Fig. 2: eine Draufsicht einer bevorzugten Ausführung der Absaugungsplatte und
- Fig. 3: eine Ausschnittdarstellung einer gegenüber Fig. 1 modifizierten Vorrichtung.

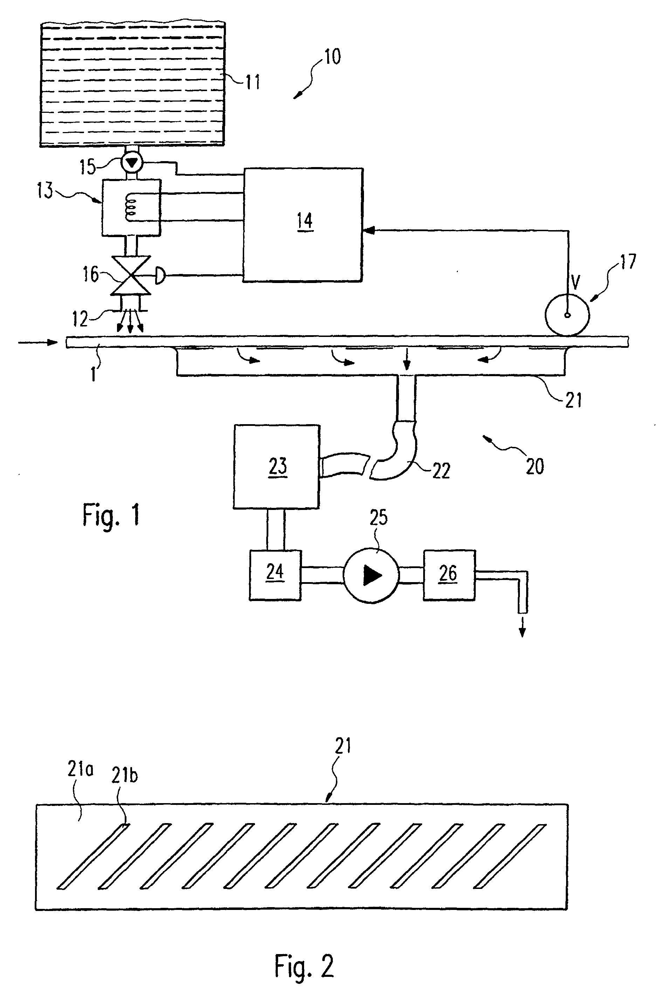

Die in Fig. 1 schematisch dargestellte Vorrichtung zur Messung organischer und/oder anorganischer Begleitfrachten in einem Textilgut 1 umfaßt als Grundkomponenten eine Aufbringeinrichtung 10 für ein Heißdampf-Wasser-Gemisch und eine Absammeleinrichtung 20 zum Aufnehmen und zur Untersuchung des Heißdampf-Wasser-Gemischs nach Durchgang durch das Textilgut 1.

Die Aufbringeinrichtung 10 umfaßt einen Wasserbehälter 11, aus dem einer Aufspritzdüse 12 mittels einer Pumpe 15 Wasser zugeführt wird, nachdem es einen Durchlauferhitzer 13, in dem es erhitzt und teilweise verdampft wird, und ein steuerbares Ventil 16 zur Einstellung des Volumenstromes passiert hat. Über eine Steuereinrichtung 14 werden die Pumpe 15, der Durchlauferhitzer 13 und das steuerbare Ventil 16 gesteuert, und zwar insbesondere in Abhängigkeit von der Fördergeschwindigkeit des Textilgutes 1, die mittels eines Drehmelders 17 erfaßt wird, der auf dem durchlaufenden Textilgut 1 läuft. Über den Drehmelder 17 wird auch ein etwaiger Stillstand des Textilgutes 1 erfaßt, und das entsprechende Signal wird in der Steuereinrichtung 14 in ein Stellsignal zum Schließen des Ventils 16 und/oder zur Abschaltung der Pumpe 15 verarbeitet.

Auf der der Düse 12 gegenüberliegenden Seite des Textilgutes 1 befindet sich, zur Düse 12 in Förderrichtung des Textilgutes 1 um einen vorbestimmten Abstand versetzt, eine Absaugplatte 21, durch die das in das Textilgut 1 eingebrachte Heißdampf-Wasser-Gemisch mittels einer Pumpe 22 über einen Verbindungsschlauch 22 abgesaugt wird. Der Verbindungsschlauch 22 ist mit einem Eingang eines Kühlers (Kondensators) 23 verbunden, der seinerseits ausgangsseitig mit einem Filter 24 verbunden ist. Nach Durchlaufen des Kondensators 23 und des Filters 24 gelangt das abgesammelte Wasser in eine Meßzelle 26, wo - in an sich bekannter Weise - eine qualitative und/oder quantitative Analyse, mittels beispielsweise pH- bzw. ionensensitiver Elektroden-Leitfähigkeitssensoren, eines Flammphotometers, eines Gaschromatographen o.ä., ausgeführt wird.

Die Aufspritzdüse 12 hat einen vorbestimmten Abstand zum Textilgut 1, der in jedem Falle das Auftreten einer Bremswirkung und eine damit verbundene Deformation des Textilgutes ausschließt, und auch zwischen der Unterseite des Textilgutes 1 und der Absaugplatte 21 wird bevorzugt ein (sehr kleiner) Abstand eingehalten. Da zudem die Absaugplatte 21 aus oberflächenglasierter Keramik gefertigt ist, welche gegenüber dem Textilgut 1 einen äußerst niedrigen Reibungskoeffizienten aufweist, ist das Auftreten einer Bremswirkung auch an der Unterseite der Textilbahn praktisch ausgeschlossen.

Der horizontale Abstand zwischen der Aufspritzdüse 12 und der Absaugplatte 21 ist so gewählt, daß das aufgespritzte Heißdampf-Wasser-Gemisch das Textilgut 1 bis zur Unterseite hin durchsetzen kann, bevor es zum ersten Schlitz der Absaugplatte gelangt und dann dem dort wirkenden Unterdruck (insbesondere unterhalb 200 mbar) unterliegt. Die Ausbildung der Aufspritzdüse 12 ist derart, daß ein relativ kleiner Flächenbereich des Textilgutes, dessen Breite auf die Breite der Absaugfläche (vergleiche die nachfolgende Beschreibung zu Fig. 2) abgestimmt ist, durchfeuchtet wird. In einer hier nicht dargestellten Ausführung ist die Düse 12 in ihrem Abstand zur Absaugplatte 21 und/oder in ihrer Höhe über der Textilbahn 1 verstellbar positioniert, womit eine Abstimmung des Aufbringvorganges auf die speziellen Eigenschaften des Textilgutes und dessen Dicke sowie auf die Fördergeschwindigkeit erfolgen kann. Zusätzliche (hier ebenfalls nicht gezeigte) Einstell- bzw. Programmierungsmittel ermöglichen zudem auch eine Einstellung der aufgespritzten Lösungsmittelmenge nicht nur von der über den Drehmelder 17 erfaßten Fördergeschwindigkeit, sondern auch in Abhängigkeit von den Eigenschaften des Textilgutes.

In Fig. 2 ist in einer Draufsicht die Absaugplatte 21 gezeigt. Es ist zu erkennen, daß sie in einem annähernd rechteckigen Grundkörper 21a eine Mehrzahl von parallel zueinander und in Förderrichtung hintereinander angeordneten, zur Förderrichtung unter einem Winkel von 45° schräg gestellten Absaugschlitzen 21b aufweist. Durch die hier gezeigte Ausbildung der Absaugplatte wird zum einen eine relativ große effektive Absaugfläche realisiert und zum anderen - durch deren Aufteilung in eine Mehrzahl von kleinen Teilflächen, die insbesondere in Förderrichtung jeweils nur eine kleine Erstreckung haben - ein Anund Einsaugen des Textilgutes wirkungsvoll verhindert.

Die in Fig. 2 gezeigte spezielle Ausbildung wird derzeit als bevorzugt angesehen; grundsätzlich sind aber auch andere Neigungswinkel der Absaugschlitze relativ zur Förderrichtung, bis hin zu einem Winkel von 90° bzw. Querstellung der Schlitze relativ zur Förderrichtung, möglich.

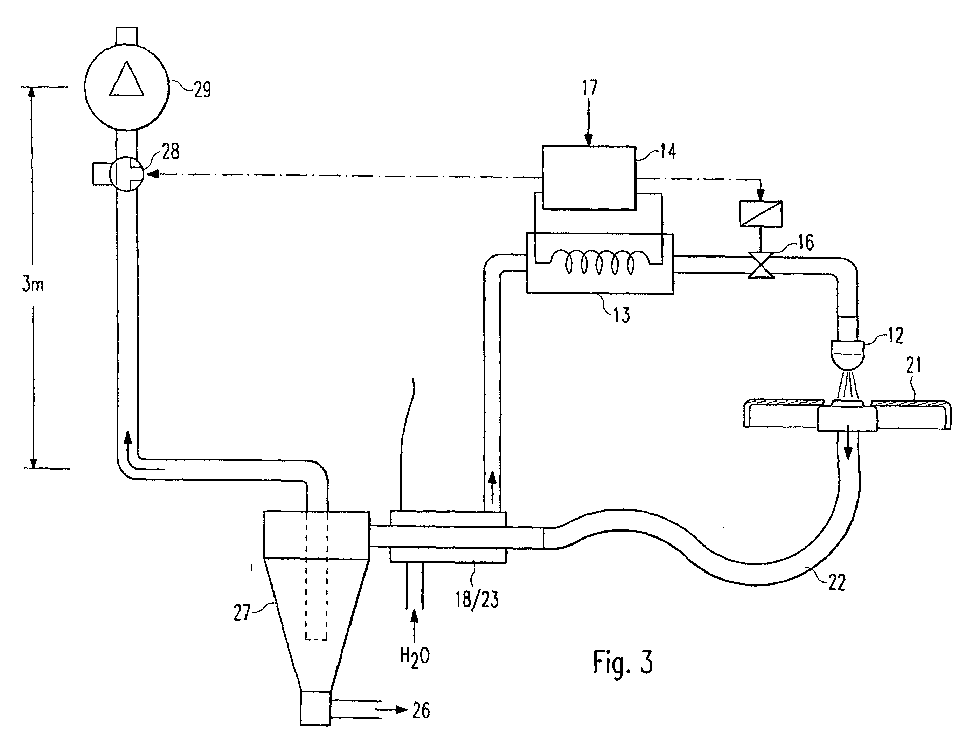

In Fig. 3 ist eine modifizierte Ausführung der Gesamtanordnung dargestellt, wobei die mit Fig. 1 übereinstimmenden Bezugsziffern gleiche Komponenten bezeichnen und diese hier nicht nochmals erläutert werden.

Eine wesentliche Fortbildung gegenüber der in Fig. 1 gezeigten Vorrichtung besteht darin, daß entionisiertes Wasser H₂O über einen Gegenstromkühler 18/23, der die Funktionen eines Vorheizers für das aufzuspritzende Wasser und eines Kondensators für das aufgesammelte Heißdampf-Wasser-Gemisch in energiesparender Weise in sich vereinigt, vor-erwärmt und erst dann dem Durchlauferhitzer 13 zugeführt wird.

Die zweite wesentliche Besonderheit besteht im Vorsehen eines dem Gegenstromkühler 18/23 nachgeschalteten Zyklonabscheiders 27, in den einerseits die Absaugleitung 22 und andererseits ein mit einem Absperrventil 28 und einem Seitenkanalgebläse 29 versehener Seitenkanal mündet und dessen Auslauf (über die in Fig. 1 gezeigten weiteren Komponenten) schließlich mit der Meßzelle 26 verbunden ist.

Die Steuereinrichtung 14 ist - wie durch die strichpunktierten Linien angedeutet - hier derart ausgebildet, daß sie bei Empfang eines den Stillstand des Textilgutes kennzeichnenden Signals vom Drehmelder 17 einerseits ein Steuersignal an das Absperrventil 28, das dessen Schließung veranlaßt, und andererseits ein Steuersignal an das steuerbare Ventil 16 ausgibt, das die Schließung desselben bewirkt. Somit wird bei einem Stillstand der Textilbahn sowohl die weitere Aufspritzung von Heißdampf-Wasser-Gemisch unterbunden als auch die Absaugung abgeschaltet, so daß die Aufnahme verfälschender Meßergebnisse in der Meßzelle 26 von vornherein unterbunden wird.

Die Ausführung ist nicht auf die oben beschriebenen Beispiele beschränkt, sondern ebenso in einer Vielzahl von Abwandlungen möglich. Derartige Abwandlungen betreffen sowohl das Material als auch (wie oben bereits erwähnt) die spezielle Geometrie der Absaugplatte und auch das Vorsehen spezieller Einstell- und Steuermöglichkeiten bzw. - in besonders einfacher und kostengünstiger Ausführung - den Verzicht auf solche.

### Bezugszeichenliste

- 1: Textilgut
- 10: Aufbringeinrichtung
- 11: Wasserbehälter
- 12: Aufspritzdüse
- 13: Durchlauferhitzer
- 14: Steuereinrichtung
- 15: Pumpe
- 16: Steuerbares Ventil
- 17: Drehmelder
- 18: Vorheizer
- 20: Absammeleinrichtung
- 21: Absaugplatte
- 22: Pumpe
- 23: Kühler (Kondensator)
- 24: Filter
- 25: Absaugpumpe
- 26: Meßzelle
- 27: Zyklonabscheider
- 28: Absperrventil
- 29: Seitenkanalgebläse

## Patentansprüche

1. Verfahren zur Messung organischer und/oder anorganischer Begleitfrachten im Inneren eines flüssigkeitsdurchlässigen flächigen Gutes, insbesondere eines Textilgutes (1), zwischen dessen erster und zweiter Oberfläche, mit den Schritten:
- Aufbringen eines Lösungsmittels (H₂O) auf das Gut von der ersten Oberfläche her,
- Absammeln des Lösungsmittels mit der darin enthaltenen Begleitfracht von der zweiten Oberfläche des Gutes,
- qualitative und/oder quantitative Bestimmung der Begleitfracht in einer Analyseneinrichtung (26),
wobei das Aufbringen und Absammeln des Lösungsmittels amin einer Förderrichtung durchlaufenden Gut erfolgt und
das Aufbringen aus einer Düse (12) mit Abstand zum durchlaufenden Gut (1) in einem ersten Raumbereich und das Absammeln mittels einer Absaugplatte (21), über die das Gut frei läuft, in einem zweiten, vom ersten insbesondere beabstandeten, Raumbereich in Förderrichtung stromabwärts des ersten Raumbereiches ausgeführt wird.

2. Verfahren nach Anspruch 1,
**dadurch gekennzeichnet, daß**
das Lösungsmittel dampfförmig ist oder Dampf enthält und das abgesammelte Lösungsmittel gekühlt und kondensiert (23) wird und das Kondensat der Analyseneinrichtung (26) zugeführt wird.

3. Verfahren nach Anspruch 2,
**dadurch gekennzeichnet, daß**
ein Wasser-Wasserdampf-Gemisch, insbesondere aus entionisierten Wasser, als Lösungsmittel aufgespritzt wird.

4. Vorrichtung zur Durchführung des Verfahrens nach einem der vorangehenden Ansprüche, mit
- einer Transporteinrichtung, die das Gut in einer Förderrichtung transportiert,
- einer Aufbringeinrichtung (10) zum Aufbringen des Lösungsmittels, die eine Druckpumpe (15) und eine mit dieser verbundene, auf den ersten Raumbereich an der ersten Oberfläche des Gutes gerichtete Düse (12) umfaßt,
- einer Absammeleinrichtung (20), die eine Unterdruck erzeugende Pumpen- oder Gebläseeinrichtung (25; 29) und eine mit dieser verbundene Absaugplatte (21) aufweist, welche in dem zweiten Raumbereich nahe der zweiten Oberfläche des Gutes stromabwärts von ersten Raumbereich und insbesondere von diesem beabstandet, angeordnet ist und
- einer mit der Absaugplatte verbundenen Meßeinrichtung (26).

5. Vorrichtung nach Anspruch 4,
**dadurch gekennzeichnet, daß**
die Absaugplatte (21) eine Mehrzahl von in Förderrichtung hintereinander, insbesondere schräg zur Förderrichtung, angeordneten Absaugschlitzen (21b) in einem Grundkörper (21a) aufweist.

6. Vorrichtung nach Anspruch 4 oder 5,
**dadurch gekennzeichnet, daß**
die Absaugplatte (21) eine Oberfläche mit in Bezug auf das flächige Gut (1) niedrigem Reibungskoeffizienten, insbesondere eine keramische Oberfläche, aufweist.

7. Vorrichtung nach einem der Ansprüche 4 bis 6,
**dadurch gekennzeichnet, daß**
die Absammeleinrichtung (21) einen mit der Absaugplatte (21) verbundenen Kühler (23) sowie einen diesem nachgeschaltetem Zyklonabscheider (27) aufweist.

8. Vorrichtung nach einem der Ansprüche 4 bis 6 und Anspruch 7,
**dadurch gekennzeichnet, daß**
das Absauggebläse (29) ein Seitenkanalgebläse umfaßt, welches insbesondere über eine Unterdruckleitung mit dem Zyklonabscheider (27) and über diesen sowie eine Absaugleitung (22) mit der Absaugplatte (21) in Verbindung steht.

9. Vorrichtung nach einem der Ansprüche 4 bis 8,
**dadurch gekennzeichnet, daß**
der Abstand zwischen dem ersten und zweiten Raumbereich derart eingestellt ist, daß das aufgespritzte Lösungsmittel (H₂O) das Textilgut (1) vollständig durchdringen kann.

10. Vorrichtung nach Anspruch 9,
**dadurch gekennzeichnet, daß**
der Abstand zwischen der Düse (12) und der Absaugplatte (21) einstellbar ist.

11. vorrichtung nach einem der Ansprüche 4 bis 10,
**dadurch gekennzeichnet, daß**
die Aufbringeinrichtung (10) Mittel (14) zur Steuerung der Lösungsmittelmenge in Abhängigkeit von der Fördergeschwindigkeit und/oder von Eigenschaften des Gutes (1) aufweist.

12. Vorrichtung nach einem der Ansprüche 4 bis 11,
**gekennzeichnet durch**
Mittel zum Abschalten der Lösungsmittelzufuhr zur Düse (12) und zur Verringerung des Unterdruckes an der Absaugplatte (21) bei Erfassung eines Stillstandes des durchlaufenden Gutes (1) **durch** einen mit diesem in Kontakt stehenden Detektor (17).

13. Vorrichtung nach einem der Ansprüche 7 bis 12,
**dadurch gekennzeichnet, daß**
der Kühler (23) als Gegenstromkühler (18/23) ausgebildet ist, durch den als Kühlmittel das frische Lösungsmittel (H₂O) geleitet und somit vorgeheizt wird.

## Claims

1. A method of measuring organic and/or inorganic accompanying substances within a liquid-permeable planar material, in particular a textile material (1), between its first and second surfaces, with the steps of:
- applying a solvent (H₂O) to the material from the first surface,
- collecting the solvent with the accompanying substance contained therein from the second surface of the material,
- qualitative and/or quantitative determination of the accompanying substance in an analysing device (26),
wherein the application and collection of the solvent is carried out on the material passing through in a conveying direction and the application is effected from a nozzle (12) at a distance from the traversing material (1) in a first chamber zone and the collection is effected by means of a suction plate (21), over which the material passes freely, in a second chamber zone in particular spaced apart from the first chamber zone, downstream of the first chamber zone in conveying direction.

2. A method according to Claim 1, **characterised in that** the solvent is in the form of a vapour or contains vapour and the collected solvent is cooled and condensed (23), and is the condensate fed to the analysing device (26).

3. A method according to Claim 2, **characterised in that** a water-steam mixture, in particular consisting of deionised water, is sprayed on as the solvent.

4. An apparatus for carrying out the method according to any one of the preceding Claims, having
- a conveying means which conveys the material in a conveying direction,
- an application means (10) for applying the solvent, which means comprises a pressure pump (15) and, connected thereto, a nozzle (12) directed on to the first chamber zone on the first surface of the material,
- a collection means (20) which has a pump or fan means (25;29) creating an underpressure and a suction plate (21) which is connected thereto and which is disposed in the second chamber zone close to the second surface of the material downstream of the first chamber zone and in particular spaced apart therefrom, and
- a measuring device (26) connected to the suction plate.

5. An apparatus according to Claim 4, **characterised in that** the suction plate (21) has a plurality of suction slots (21b) in a base member (21a), which are arranged one behind the other, in particular inclined relative to the conveying direction.

6. An apparatus according to Claim 4 or 5, **characterised in that** the suction plate (21) has a surface with low coefficients of friction in relation to the planar material (1), in particular a ceramic surface.

7. An apparatus according to any one of Claims 4 to 6, **characterised in that** the collection plate (21) has a cooler (23) connected to the suction plate (21) and also a centrifugal separator (27) connected downstream thereof.

8. An apparatus according to any one of Claims 4 to 6 and Claim 7, **characterised in that** the suction fan (29) comprises a side channel fan which, in particular via an underpressure line, is in communication with the cyclone separator (27) and via the latter and a suction line (22) is in communication with the suction plate (21).

9. An apparatus according to any one of Claims 4 to 8, **characterised in that** the distance between the first and second chamber zones is adjusted so that the sprayed-on solvent (H₂O) can fully penetrate the textile material (1).

10. An apparatus according to Claim 9, **characterised in that** the distance between the nozzle (12) and the suction plate (21) is adjustable.

11. An apparatus according to any one of Claims 4 to 10, **characterised in that** the application means (10) has means (14) for controlling the amount of solvent as a function of the conveyor speed and/or of properties of the material (1).

12. An apparatus according to any one of Claims 4 to 11, **characterised by** means for shutting off the supply of solvent to the nozzle (12) and for reducing the underpressure at the suction plate (21) when a sensor (17) in contact with the traversing material (1) detects a stoppage thereof.

13. An apparatus according to any one of Claims 7 to 12, **characterised in that** the cooler (23) is in the form of a countercurrent cooler (18/23), through which the fresh solvent (H₂O) is directed as coolant and is thus preheated.

## Revendications

1. Procédé pour mesurer des substances d'accompagnement organiques et/ou inorganiques à l'intérieur d'un produit plat perméable aux liquides, en particulier d'un textile (1), entre sa première et sa seconde surfaces, présentant les étapes qui consistent à :
- appliquer un diluant (H₂O) sur le produit, à partir de la première surface,
- collecter le diluant avec la substance d'accompagnement contenue dedans, à partir de la seconde surface du produit,
- déterminer qualitativement et/ou quantitativement la substance d'accompagnement dans un dispositif d'analyse (26),
dans lequel l'application et la collecte du diluant s'opère sur le produit circulant dans un sens de transport et l'application est réalisée dans une première zone spatiale à partir d'une buse (12) à distance du produit (1) circulant, et la collecte s'opère dans une seconde zone spatiale, en particulier à distance de la première, dans le sens d'écoulement en aval de la première zone spatiale, au moyen d'une plaque d'aspiration (21) au-dessus de laquelle le produit circule librement.

2. Procédé selon la revendication 1,
**caractérisé en ce que**
le diluant est à l'état de vapeur ou contient de la vapeur et le diluant collecté est refroidi ou condensé (23) et le condensat est acheminé vers le dispositif d'analyse (26).

3. Procédé selon la revendication 2,
**caractérisé en ce que**
un mélange eau/vapeur d'eau, en particulier d'eau désionisée, est pulvérisé en tant que diluant.

4. Dispositif pour mettre en oeuvre le procédé selon l'une des revendications précédentes, comportant
- un système de transport, qui transporte le produit dans un sens de transport,
- un dispositif d'application (10) pour appliquer le diluant, qui comprend une pompe de pression (15) et, reliée à celle-ci, une buse orientée vers la première zone spatiale sur la première surface du produit,
- un dispositif de collecte (20), qui présente un dispositif à pompe ou soufflante (25 ; 29) créant une dépression et, reliée à celui-ci, une plaque d'aspiration (21) laquelle est disposée dans la seconde zone spatiale à proximité de la seconde surface du produit, en aval de la première zone spatiale et en particulier à distance de celle-ci et
- un dispositif de mesure (26) relié à la plaque d'aspiration.

5. Dispositif selon la revendication 4,
**caractérisé en ce que**
la plaque d'aspiration (21) présente dans un corps de base (21a) une pluralité de fentes d'aspiration (21b) disposées les unes derrière les autres dans le sens de transport, en particulier obliquement par rapport au sens de transport.

6. Dispositif selon la revendication 4 ou 5,
**caractérisé en ce que**
la plaque d'aspiration (21) présente une surface à faible coefficient de frottement par rapport au produit plat (1), en particulier une surface en céramique.

7. Dispositif selon l'une des revendications 4 à 6,
**caractérisé en ce que**
le dispositif de collecte (21) présente, relié à la plaque d'aspiration (21), un refroidisseur (23) ainsi qu'un séparateur cyclone (27) monté en aval de celui-ci.

8. Dispositif selon l'une des revendications 4 à 6 et la revendication 7,
**caractérisé en ce que**
la soufflante d'aspiration (29) comprend une soufflante à canal latéral laquelle, en particulier via une conduite à dépression, est en liaison avec le séparateur cyclone (27) et par celui-ci ainsi que via une conduite d'aspiration (22) avec la plaque d'aspiration (21).

9. Dispositif selon l'une des revendications 4 à 8,
**caractérisé en ce que**
la distance entre la première et la seconde zones spatiales est définie de telle façon que le diluant vaporisé (H₂O) peut traverser totalement le produit textile (1).

10. Dispositif selon la revendication 9,
**caractérisé en ce que**
la distance entre la buse (12) et la plaque d'aspiration (21) est réglable.

11. Dispositif selon l'une des revendications 4 à 10,
**caractérisé en ce que**
le dispositif d'application (10) présente des moyens (14) pour commander la quantité de diluant en fonction de la vitesse de transport et/ou des caractéristiques du produit (1).

12. Dispositif selon l'une des revendications 4 à 11,
**caractérisé par**
des moyens pour arrêter l'arrivée de diluant vers la buse (12) et pour réduire la dépression à la plaque d'aspiration (21) lorsque l'arrêt du produit circulant (1) est détecté par un détecteur (17) en contact avec celui-ci.

13. Dispositif selon l'une des revendications 7 à 12,
**caractérisé en ce que**
le refroidisseur (23) est réalisé en tant que refroidisseur à contre-courant (18/23) par lequel le diluant frais (H₂O) est acheminé en tant que produit de refroidissement et ainsi préchauffé.
